# EUROPÄISCHE PATENTANMELDUNG

(11) **EP 2 567 630 A1**
(43) Veröffentlichungstag der Anmeldung: **13.03.2013**
(21) Anmeldenummer: 11180910.9
(22) Anmeldetag: 12.09.2011
(51) Int. Cl.: A42B 3/04, A61F 9/02

(54) **Tragbare Schutzvorrichtung und Verfahren zur Zugangskontrolle mit Hilfe einer tragbaren Schutzvorrichtung**

(71) Anmelder: Siemens Aktiengesellschaft, 80333 München (DE)
(72) Erfinder: Falk, Rainer, 85435 Erding (DE)

(57) **Zusammenfassung**

Eine Schutzbrille oder ein Schutzhelm, welche zum Führen von Zweirädern oder in industriellen Umgebungen ohnehin getragen werden müssen, werden mit einem Transponder ausgerüstet, um den Schutzhelm beziehungsweise die Schutzbrille als Authentisierungs-Token zur vereinfachten Zugangskontrolle nutzbar zu machen. Vorzugsweise erfolgt eine biometrische Authentisierung des Trägers durch die Schutzvorrichtung. Dadurch kann diese nicht missbräuchlich durch andere Nutzer verwendet werden.

## Beschreibung

Die Erfindung betrifft eine tragbare Schutzvorrichtung, beispielsweise eine Schutzbrille oder einen Schutzhelm, welche in industriellen Umgebungen, bei Arbeiten an oder mit Maschinen oder beim Fahren von Zweirädern zur Sicherheit getragen werden muss. So ist das Tragen von Schutzvorrichtungen in industriellen Umgebungen wie Fertigungshallen, Raffinerien, Werften, Kraftwerken oder Stahlwerken vorgeschrieben.

Die Erfindung betrifft weiterhin eine Authentisierung von berechtigten Nutzern, welche Zugang zu Räumen, zu Gebäuden oder zur Bedienung von technischen Anlagen, oder auch zum Führen von Fahrzeugen erhalten sollen. Durch die Authentisierung wird hier eine unberechtigte Nutzung beziehungsweise ein unberechtigter Zugang verhindert.

Zur Authentisierung von berechtigten Nutzern sind unterschiedliche Verfahren bekannt. Beispielsweise kann ein berechtigter Nutzer ein Passwort auf einer Tastatur eingeben. Weiterhin ist eine biometrische Authentisierung durch Erkennung einer Handfläche, eines Gesichts oder einer Stimme bekannt. Ebenfalls bekannt ist eine Authentisierung mittels einer Zugangskarte oder eines Firmenausweises, die entweder in ein Lesegerät eingeführt werden oder über einen Funktransponder verfügen.

Aus der DE 2008 050 610 A1 ist ein Schutzhelm bekannt, welcher mit einem Transponder beziehungsweise mit einem Identifikationssystem ausgerüstet ist. Dies dient in erster Linie einer Lokalisierung eines Trägers des Schutzhelms.

Aus der EP 2 087 802 A2 ist ein Schutzhelm mit drahtloser Datenübertragung bekannt. Der Schutzhelm ist hierzu mit einem Transponder ausgerüstet, welcher einem Träger des Helms eine charakteristische Identitätsnummer zuordnet. Weiterhin verfügt der Schutzhelm über Sensoren zur Messung humaner Werte wie Temperatur, Puls oder Feuchtigkeit. Dies dient der Überwachung eines Gesundheitszustandes des Helmträgers. Bei gesundheitlichen Problemen kann ein Alarm ausgelöst werden.

Aus dem Dokument "Eingebettete, vernetzte und autonom handelnde Computersysteme: Szenarien und Visionen", erhältlich im Internet am 01. September 2011 unter http://www.inf.usi.ch/faculty/langheinrich/articles/papers/ma ttern-taswiss-szenarien.pdf, ist bekannt, eine Maschine nur dann zu aktivieren, wenn ein Bediener eine Schutzvorrichtung trägt. So kann die Maschine nur dann genutzt werden, wenn die erforderliche Sicherheitsausrüstung durch den Bediener getragen wird. Als Beispiel ist dort eine Bohrmaschine offenbart, welche nur mit aufgesetztem Schutzhelm und Schutzbrille betrieben werden kann.

Aus der US 2009/0058712 A1 ist bekannt, an Schutzhelmen für einen Kampfeinsatz eine Freund-Feind-Authentisierungseinheit zu montieren, sodass im Gefecht eigene und fremde Truppen unterscheidbar sind. Hierbei werden Infrarot-Signale verwendet, um eine Challenge-Response-Authentisierung durchzuführen. Die Authentisierungseinheit verfügt über einen Kontrollmechanismus mittels biometrischer Messungen, um eine Nutzung einer in Feindeshand geratenen Authentisierungseinheit zu verhindern. Hierzu wertet ein biometrischer Sensor einen Fingerabdruck des Nutzers aus, bevor die Authentisierungseinheit aktiviert wird.

Der Erfindung liegt die Aufgabe zugrunde, eine tragbare Schutzvorrichtung und ein Verfahren zur Zugangskontrolle mit Hilfe einer tragbaren Schutzvorrichtung anzugeben, welche eine Alternative zum Stand der Technik bereitstellen und eine zuverlässige Nutzerauthentisierung auch in industriellen Einsatzumgebungen ermöglichen.

Diese Aufgabe wird erfindungsgemäß durch eine tragbare Schutzvorrichtung gelöst, welche mit einer drahtlosen Kommunikationsschnittstelle ausgerüstet ist. Die tragbare Schutzvorrichtung ist durch ein Authentisierungsmodul gekennzeichnet, welches für eine Authentisierung eines Trägers der tragbaren Schutzvorrichtung unter Nutzung der drahtlosen Kommunikationsschnittstelle eingerichtet ist.

Bei dem Verfahren zur Zugangskontrolle mit Hilfe einer tragbaren Schutzvorrichtung ist diese mit einer drahtlosen Kommunikationsschnittstelle ausgerüstet. Das Verfahren ist dadurch gekennzeichnet, dass die tragbare Schutzvorrichtung ein Authentisierungsmodul aufweist, welches für eine Authentisierung eines Trägers der tragbaren Schutzvorrichtung unter Nutzung der drahtlosen Kommunikationsschnittstelle eingerichtet ist. Das Authentisierungsmodul überträgt hierzu mit Hilfe der drahtlosen Kommunikationsschnittstelle eine Nutzerauthentisierungsinformation, welche insbesondere durch eine kryptographische Prüfinformation geschützt wird, an ein Zugangskontrollsystem, insbesondere für einen Aufenthalt in einer Produktionsstätte, eine Wartung einer Maschine, einen Durchlass durch eine Tür oder einer Nutzung eines Fahrzeugs. Weiterhin überprüft das Zugangskontrollsystem die Nutzerauthentisierungsinformation. Abschließend schaltet das Zugangskontrollsystem eine Funktionalität frei, sofern der Träger als zugelassener Nutzer erkannt wurde.

Die tragbare Schutzvorrichtung stellt so eine einfach verwendbare Nutzerauthentisierung bereit, welche sich besonders für industrielle Einsatzumgebungen oder Zweiräder eignet, da dort ohnehin ein Schutzhelm oder eine Schutzbrille getragen werden müssen. Die tragbare Schutzvorrichtung wird somit zu einem Authentisierungs-Token beziehungsweise Zugangs-Token für den Träger.

Die Nutzung der tragbaren Schutzvorrichtung zur Authentisierung hat darüber hinaus weitere Vorteile. Beispielsweise entfällt die Notwendigkeit, mit Hilfe einer Tastatur oder eines Ziffernfeldes ein Passwort beziehungsweise einen Zifferncode einzutippen. Dies ist gerade in industriellen Umgebungen praktisch, wenn Handschuhe getragen werden oder die Hände des Nutzers schmutzig sind. Auch eine biometrische Authentisierung über Scannen einer Handfläche, eine Gesichtserkennung oder eine Stimmerkennung ist in einer verschmutzten oder lauten Industrieumgebung problematisch. Ein an einem Band hängender Transponder zur Authentisierung ist in einer solchen Umgebung ebenfalls unpraktisch, da das Band an einer Maschine hängen bleiben kann. Würde der Transponder unter einer Schutzkleidung des Nutzers getragen, so würde dies die Funkübertragung verschlechtern. Auch könnte der Nutzer dann vergessen, seinen Transponder einzustecken. Dem gegenüber bietet die tragbare Schutzvorrichtung gerade in der zuvor beschriebenen Industrieumgebung offensichtliche Vorteile.

Gemäß einer Weiterbildung ist die tragbare Schutzvorrichtung als Schutzhelm oder Schutzbrille ausgestaltet. Dies ist von Vorteil, da Schutzhelme oder Schutzbrillen in industriellen Umgebungen oder beim Führen von Zweirädern ohnehin getragen werden müssen. Die jeweiligen Schutzvorrichtungen sind dabei oft einem bestimmten Nutzer zugeordnet.

Gemäß einer Ausführungsform ist die drahtlose Kommunikationsschnittstelle ein RFID-Transponder, ein WLAN-Port oder ein Infrarot-Port.

In einer Weiterbildung ist die tragbare Schutzvorrichtung mit mindestens einem Neigungssensor und/oder Drucksensor ausgerüstet, welcher mit dem Authentisierungsmodul verbunden oder in dieses eingebaut ist. Das Authentisierungsmodul ist zur Detektion eines Tragens der tragbaren Schutzvorrichtung anhand von Signalen des mindestens einen Neigungssensors oder Drucksensors eingerichtet. Diese Weiterbildung hat den Vorteil, dass bei der Authentisierung sichergestellt werden kann, dass die tragbare Schutzvorrichtung auch tatsächlich getragen wird. Hierdurch wird die Arbeitssicherheit erhöht. Gemäß einer Ausführungsform ist die tragbare Schutzvorrichtung mit Sensoren ausgerüstet. Dabei handelt es sich insbesondere um mechanische Sensoren, kapazitive Sensoren, Ultraschall-Abstandssensoren und/oder mindestens eine UltraschallSonde, welche für eine Erfassung einer Schädelform, einer Oberflächenbeschaffenheit, einer Kontur und/oder einer Anatomie eines Kopfes des Trägers ausgelegt sind. Alternativ handelt es sich bei den Sensoren um mindestens zwei Elektroden, welche für eine Erfassung eines Elektrokardiogramms, eines Elektro-Okulogramms oder eines Elektroenzephalogramms des Trägers ausgelegt sind. Weiterhin kann es sich bei den Sensoren auch um einen Iris-Scanner oder einen Retina-Scanner handeln. Die Sensoren sind mit dem Authentisierungsmodul verbunden oder in dieses eingebaut. Das Authentisierungsmodul ist für eine biometrische Authentisierung des Trägers anhand von Signalen der Sensoren eingerichtet.

In einer Weiterbildung des Verfahrens führt das Authentisierungsmodul nach einem erstmaligen Aufsetzen oder nach einem zwischenzeitlichen Abnehmen und erneuten Aufsetzen der tragbaren Schutzvorrichtung durch den Träger eine Erst-Authentisierung des Trägers mit Hilfe einer gesonderten Authentisierungsvorrichtung durch. Das Authentisierungsmodul führt die Authentisierung des Trägers nur durch, wenn die Erst-Authentisierung erfolgreich war.

In einer Weiterbildung werden bei der Erst-Authentisierung biometrische Referenzdaten durch die tragbare Schutzvorrichtung gemessen oder von der gesonderten Authentisierungsvorrichtung übertragen. Die biometrischen Referenzdaten werden im Authentisierungsmodul gespeichert.

Gemäß einer Ausführungsform ermittelt das Authentisierungsmodul zur Authentisierung des Trägers aktuelle biometrische Daten. Das Authentisierungsmodul führt die Authentisierung nur durch, wenn eine Abweichung der aktuellen biometrischen Daten von den biometrischen Referenzdaten einen Schwellwert unterschreitet.

Diese biometrische Authentisierung des Trägers hat den Vorteil, dass die Schutzvorrichtung eines Werkleiters nicht missbräuchlich durch andere Nutzer verwendet werden kann, um die entsprechenden Zugangsrechte zu erhalten. Die biometrische Authentisierung des Trägers stellt somit einen hohen Schutz vor Missbrauch bereit.

Auf dem computerlesbaren Datenträger ist ein Computerprogramm gespeichert, welches das Verfahren ausführt, wenn es in einem Computer abgearbeitet wird. Das Computerprogramm wird in einem Computer abgearbeitet und führt dabei das Verfahren aus.

Im Folgenden werden Ausführungsbeispiele der Erfindung anhand von Figuren näher erläutert. Es zeigen:
- Fig. 1: eine Nutzerauthentisierung mit Hilfe eines Schutzhelms,
- Fig. 2: eine Nutzerauthentisierung mit Hilfe einer Schutzbrille.

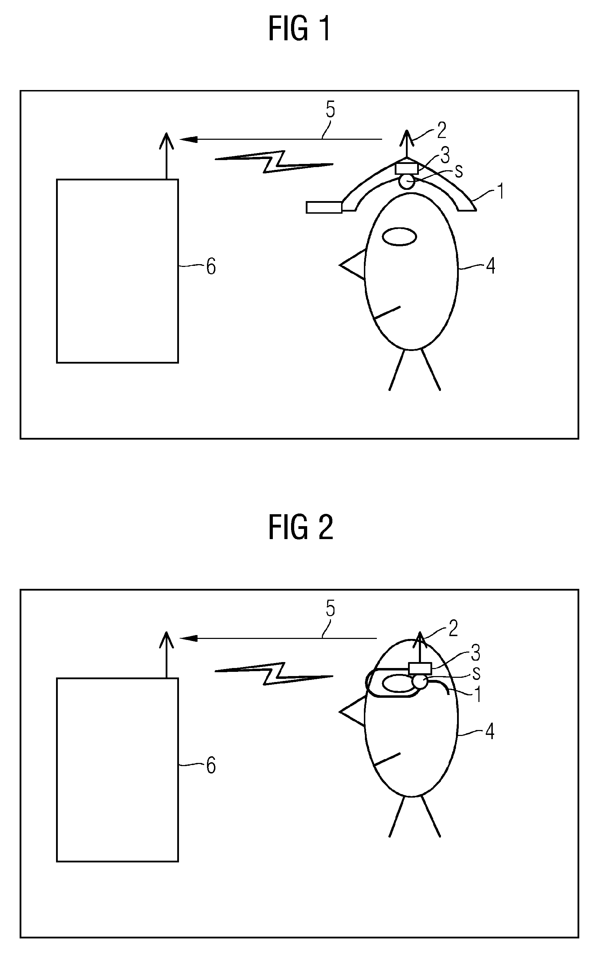

Figur 1 zeigt eine tragbare Schutzvorrichtung 1, welche hier als Schutzhelm ausgebildet ist. Die tragbare Schutzvorrichtung 1 verfügt über eine drahtlose Kommunikationsschnittstelle 2, beispielsweise ein RFID-Transponder (aktiv, semi-aktiv oder passiv), ein WLAN-Port oder ein Infrarot-Port. Sie verfügt weiterhin über einen Sensor S, welcher beispielsweise ein Neigungssensor oder Drucksensor ist und zur Detektion eines Tragens der tragbaren Schutzvorrichtung 1 eingerichtet ist.

Alternativ handelt es sich bei dem Sensor S gegebenenfalls auch um einen oder mehrere Sensoren, welche als mechanische Sensoren, kapazitive Sensoren, Ultraschall-Abstandssensoren oder Ultraschall-Sonden ausgeführt sind und eine Erfassung einer Schädelform, einer Oberflächenbeschaffenheit (Haare oder Haut), einer Kontur oder einer Anatomie eines Kopfes eines in Figur 1 gezeigten Trägers 4 der tragbaren Schutzvorrichtung 1 ermöglichen. Bei den Sensoren S kann es sich auch um zwei oder mehr Elektroden handeln, welche für eine Erfassung eines Elektrokardiogramms, eines Elektro-Okulogramms oder eines Elektroenzephalogramms des Trägers 4 ausgelegt sind. Alternativ bietet sich als Sensor S auch ein Iris-Scanner oder ein Retina-Scanner an.

Der Sensor S beziehungsweise die Sensoren S sind mit einem Authentisierungsmodul 3 verbunden oder in dieses eingebaut. Das Authentisierungsmodul 3 ist für eine biometrische Authentisierung des Trägers 4 anhand von Signalen der Sensoren S eingerichtet und verfügt hierzu beispielsweise über einen entsprechend programmierten Mikroprozessor.

Der Sensor S bietet den Vorteil, dass eine Authentisierung mit Hilfe der tragbaren Schutzvorrichtung 1 nur bei ordnungsgemäß aufgesetzter Schutzvorrichtung 1 aktiviert werden kann. Dies erhöht die Arbeitssicherheit. Denn durch einen Neigungssensor oder Drucksensor kann das Authentisierungsmodul 3 einfach erkennen, ob die tragbare Schutzvorrichtung 1 aufgesetzt ist.

Im Folgenden wird eine biometrische Authentisierung des Trägers 4 durch das Authentisierungsmodul 3 als eine Variante des Ausführungsbeispiels beschrieben. Hierbei wird als biometrische Charakteristik des Trägers 4 die geometrische Form seines Kopfes beziehungsweise Schädels erfasst. Alternativ können die Oberflächenbeschaffenheit des Kopfes, d.h. auf welchen Flächen Kopfhaar vorhanden ist, oder Sensorwerte wie Herzschlag oder Blutdruck des Trägers 4 ausgewertet werden. Auch eine Iris oder Retina des Trägers 4 kann zur biometrischen Authentisierung sensorisch erfasst werden. Die biometrische Authentisierung bietet den besonderen Vorteil, dass zu jedem Zeitpunkt überprüft werden kann, ob die tragbare Schutzvorrichtung 1 auch tatsächlich von der richtigen Person getragen wird.

Die tragbare Schutzvorrichtung 1 sendet eine Nutzerauthentisierungsinformation 5 mit Hilfe der drahtlosen Kommunikationsschnittstelle 2 an ein Zugangskontrollsystem 6. Das Zugangskontrollsystem 6 regelt beispielsweise den Zugang zu einer Fertigungshalle, einer Raffinerie, einer Werft, einem Kraftwerk oder einem Stahlwerk. Das Zugangskontrollsystem 6 kann auch eine Bedienung bestimmter Maschinen, eine Wartung technischer Anlagen oder den Betrieb eines Zweirads freischalten.

Es wurde in Bezug auf Figur 1 vorrangig auf die gegenständlichen Merkmale der tragbaren Schutzvorrichtung 1 eingegangen. Diese Ausführungen gelten analog auch für das in Figur 2 gezeigte Ausführungsbeispiel. Darüber hinaus wird für Figur 2 im Folgenden der Ablauf des Verfahrens detailliert beschrieben. Diese Ausführungen gelten jedoch auch analog für den in Figur 1 gezeigten Schutzhelm.

Figur 2 zeigt erneut eine tragbare Schutzvorrichtung 1, welche hier als Schutzbrille ausgeführt ist. Die Schutzbrille eignet sich besonders in einem chemischen Labor oder bei handwerklichen Vorgängen wir Bohren oder Fräsen. Sie verfügt ebenfalls über Sensoren S, welche erfassen, ob die Schutzbrille korrekt aufgesetzt ist.

Auch hier sieht ein erweitertes Ausführungsbeispiel biometrische Sensoren S vor, welche den Träger der Schutzbrille biometrisch authentisieren können.

Auch bei dem in Figur 2 gezeigten Ausführungsbeispiel sendet ein Authentisierungsmodul 3 mit Hilfe einer drahtlosen Kommunikationsschnittstelle 2 eine Nutzerauthentisierungsinformation 5 an ein Zugangskontrollsystem 6. Die Nutzerauthentisierungsinformation 5 wird hierbei vorzugsweise mittels einer kryptographischen Prüfinformation geschützt. Beispielsweise werden als Nutzerauthentisierungsinformation ein Benutzer-Identifikator sowie eine kryptographische Prüfsumme übertragen. Die Nutzerauthentisierungsinformation 5 wird vom Zugangskontrollsystem 6 empfangen und geprüft. Sofern im Träger 4 ein zugelassener Nutzer erkannt wird, wird eine Funktionalität freigeschaltet, das heißt es wird dem Träger 4 Zugang gewährt.

In einer Variante des Ausführungsbeispiels führt das Authentisierungsmodul 3 nach jedem Aufsetzen der tragbaren Schutzvorrichtung 1 durch den Träger 4 eine Erst-Authentisierung des Trägers 4 mit Hilfe einer gesonderten Authentisierungsvorrichtung durch. Bei der gesonderten Authentisierungsvorrichtung handelt es sich beispielsweise um ein herkömmliches Sicherheits-Token wie etwa einen Firmenausweis. Das Authentisierungsmodul 3 führt die Authentisierung (gemeint ist hier die eigentliche Authentisierung für das Zugangskontrollsystem 6 zu einem späteren Zeitpunkt) des Trägers 4 nur durch, wenn die Erst-Authentisierung erfolgreich war.

Vorzugsweise werden bei der Erst-Authentisierung biometrische Referenzdaten durch die tragbare Schutzvorrichtung 1 gemessen oder von der gesonderten Authentisierungsvorrichtung übertragen. Die biometrischen Referenzdaten werden daraufhin im Authentisierungsmodul 3 gespeichert.

Das Authentisierungsmodul 3 ermittelt in diesem Fall zur Authentisierung des Trägers 4 aktuelle biometrische Daten und führt die Authentisierung nur durch, wenn eine Abweichung der aktuellen biometrischen Daten von den biometrischen Referenzdaten einen Schwellenwert unterschreitet. Die Authentisierungsfunktion der Schutzbrille bleibt somit nur freigeschaltet, solange die ermittelten biometrischen Daten hinreichend ähnlich zu den während der Erst-Authentisierung erfassten biometrischen Referenzdaten sind. Auch kann nach einem Abnehmen und erneuten Aufsetzen der Schutzvorrichtung eine erneute Erst-Authentisierung zwingend vorgeschrieben sein.

Die beschriebenen Ausführungsformen, Weiterbildungen, Ausführungsbeispiele und Varianten lassen sich frei miteinander kombinieren.

## Patentansprüche

1. Tragbare Schutzvorrichtung (1), welche mit einer drahtlosen Kommunikationsschnittstelle (2) ausgerüstet ist,
**gekennzeichnet durch**
- ein Authentisierungsmodul (3), eingerichtet für eine Authentisierung eines Trägers (4) der tragbaren Schutzvorrichtung (1) unter Nutzung der drahtlosen Kommunikationsschnittstelle (2).

2. Tragbare Schutzvorrichtung (1) nach Anspruch 1,
- ausgestaltet als Schutzhelm oder als Schutzbrille.

3. Tragbare Schutzvorrichtung (1) nach einem der vorangegangenen Ansprüche,
- bei der die drahtlose Kommunikationsschnittstelle (2) ein RFID-Transponder, ein WLAN-Port oder ein Infrarot-Port ist.

4. Tragbare Schutzvorrichtung (1) nach einem der vorangegangenen Ansprüche,
- ausgerüstet mit mindestens einem Neigungssensor und/oder Drucksensor, welcher mit dem Authentisierungsmodul (3) verbunden oder in dieses eingebaut ist, und
- bei der das Authentisierungsmodul (3) zur Detektion eines Tragens der tragbaren Schutzvorrichtung (1) anhand von Signalen des mindestens einen Neigungssensors oder Drucksensors eingerichtet ist.

5. Tragbare Schutzvorrichtung (1) nach einem der vorangegangenen Ansprüche,
- ausgerüstet mit Sensoren (S), insbesondere
- mechanischen Sensoren, kapazitiven Sensoren, Ultraschall-Abstandssensoren und/oder mindestens einer UltraschallSonde, welche für eine Erfassung einer Schädelform, einer Oberflächenbeschaffenheit, einer Kontur und/oder einer Anatomie eines Kopfes des Trägers (4) ausgelegt sind,
- mindestens zwei Elektroden, welche für eine Erfassung eines Elektrokardiogramms, eines Elektro-Okulogramms oder eines Elektroenzephalogramms des Trägers (4) ausgelegt sind, und/oder
- einem Iris-Scanner oder einem Retina-Scanner,
- bei der die Sensoren (S) mit dem Authentisierungsmodul (3) verbunden oder in dieses eingebaut sind, und
- bei der das Authentisierungsmodul (3) für eine biometrische Authentisierung des Trägers (4) anhand von Signalen der Sensoren (S) eingerichtet ist.

6. Verfahren zur Zugangskontrolle mithilfe einer tragbaren Schutzvorrichtung (1), welche mit einer drahtlosen Kommunikationsschnittstelle (2) ausgerüstet ist,
**dadurch gekennzeichnet, dass**
- die tragbare Schutzvorrichtung (1) ein Authentisierungsmodul (3) aufweist, welches für eine Authentisierung eines Trägers (4) der tragbaren Schutzvorrichtung (1) unter Nutzung der drahtlosen Kommunikationsschnittstelle (2) eingerichtet ist,
- das Authentisierungsmodul (3) mithilfe der drahtlosen Kommunikationsschnittstelle (2) eine Nutzerauthentisierungsinformation (5), welche insbesondere durch eine kryptographische Prüfinformation geschützt wird, an ein Zugangskontrollsystem (6), insbesondere für einen Aufenthalt in einer Produktionsstätte, eine Wartung einer Maschine, einen Durchlass durch eine Tür oder eine Nutzung eines Fahrzeugs, überträgt,
- das Zugangskontrollsystem (6) die NutzerAuthentisierungsinformation (5) überprüft, und
- das Zugangskontrollsystem (6) eine Funktionalität freischaltet, sofern der Träger (4) als zugelassener Nutzer erkannt wurde.

7. Verfahren nach Anspruch 6,
- bei dem das Authentisierungsmodul (3) nach einem Aufsetzen oder nach einem zwischenzeitlichen Abnehmen und erneuten Aufsetzen der tragbaren Schutzvorrichtung (1) durch den Träger (4) eine Erst-Authentisierung des Trägers (4) mithilfe einer gesonderten Authentisierungsvorrichtung (1) durchführt, und
- bei dem das Authentisierungsmodul (3) die Authentisierung des Trägers (4) nur durchführt, wenn die Erst-Authentisierung erfolgreich war.

8. Verfahren nach Anspruch 7,
- wobei bei der Erst-Authentisierung biometrische Referenzdaten durch die tragbare Schutzvorrichtung (1) gemessen oder von der gesonderten Authentisierungsvorrichtung übertragen werden, und
- bei dem die biometrischen Referenzdaten im Authentisierungsmodul (3) gespeichert werden.

9. Verfahren nach Anspruch 8,
- bei dem das Authentisierungsmodul (3) zur Authentisierung des Trägers (4) aktuelle biometrische Daten ermittelt, und
- bei dem das Authentisierungsmodul (3) die Authentisierung nur durchführt, wenn eine Abweichung der aktuellen biometrischen Daten von den biometrischen Referenzdaten einen Schwellwert unterschreitet.

10. Computerlesbarer Datenträger,
- auf dem ein Computerprogramm gespeichert ist, welches das Verfahren nach einem der Ansprüche 6 bis 9 ausführt, wenn es in einem Computer abgearbeitet wird.

11. Computerprogramm,
- welches in einem Computer abgearbeitet wird und dabei das Verfahren nach einem der Ansprüche 6 bis 9 ausführt.
